# FASCICULE DE BREVET EUROPEEN

(11) **EP 3 798 216 B1**
(45) Date de publication et mention de la délivrance du brevet: **05.03.2025**
(21) Numéro de dépôt: 20196154.7
(22) Date de dépôt: 15.09.2020
(51) Int. Cl.: C07D 413/04, C07D 413/06, C08G 61/12

(54) **PROCÉDÉ DE FABRICATION D'UN PRODUIT RÉTICULÉ**
VERFAHREN ZUR HERSTELLUNG EINES NETZARTIGEN PRODUKTS
METHOD FOR MANUFACTURING A CROSS-LINKED PRODUCT

(30) Priorité: 30.09.2019 FR 1910775
(43) Date de publication de la demande: 31.03.2021
(73) Titulaire: ArianeGroup SAS, 78130 Les Mureaux (FR); Centre National de la Recherche Scientifique, 75016 Paris (FR); Ecole Nationale Supérieure de Chimie de Montpellier, 34296 Montpellier (FR); UNIVERSITE DE MONTPELLIER, 34090 Montpellier (FR)
(72) Inventeur: TAVERNIER, Romain, 80600 Doullens (FR); GRANADO, Lérys, 11000 Carcassonne (FR); DAVID, Ghislain, 34080 Montpellier (FR); CAILLOL, Sylvain, 34090 Montpellier (FR); FOYER, Gabriel, 33185 Le Haillan (FR)
(74) Mandataire: Cabinet Beau de Loménie

(56) Documents cités:
- RITA C.S. PEREIRA ET AL: "Influence of natural substituents in the polymerization behavior of novel bio-based benzoxazines", MATERIALS TODAY COMMUNICATIONS, vol. 21, 6 September 2019 (2019-09-06) - 6 September 2019 (2019-09-06), pages 100629, XP055707570, ISSN: 2352-4928, DOI: 10.1016/j.mtcomm.2019.100629
- SEISHI OHASHI ET AL: "Synthesis and ring-opening polymerization of 2-substituted 1,3-benzoxazine: the first observation of the polymerization of oxazine ring-substituted benzoxazines", POLYMER CHEMISTRY, vol. 7, no. 46, 1 January 2016 (2016-01-01), pages 7177 - 7184, XP055626808, ISSN: 1759-9954, DOI: 10.1039/C6PY01686C
- MOHAMED BAQAR ET AL: "Methylol-functional benzoxazines as precursors for high-performance thermoset polymers: Unique simultaneous addition and condensation polymerization behavior", JOURNAL OF POLYMER SCIENCE PART A: POLYMER CHEMISTRY, vol. 50, no. 11, 1 June 2012 (2012-06-01), pages 2275 - 2285, XP055174650, ISSN: 0887-624X, DOI: 10.1002/pola.26008
- KAN ZHANG ET AL: "Polymerization of an AB-Type Benzoxazine Monomer toward Different Polybenzoxazine Networks: When Diels-Alder Reaction Meets Benzoxazine Chemistry in a Single-Component Resin", MACROMOLECULES, vol. 52, no. 19, 25 September 2019 (2019-09-25) - 25 September 2019 (2019-09-25), WASHINGTON, DC, UNITED STATES, pages 7386 - 7395, XP055707581, ISSN: 0024-9297, DOI: 10.1021/acs.macromol.9b01581
- MASAHIDE GOTO ET AL: "Synthesis and crosslinking reaction of polyacetylenes substituted with benzoxazine rings: Thermally highly stable benzoxazine resins", JOURNAL OF POLYMER SCIENCE, PART A: POLYMER CHEMISTRY, vol. 56, no. 16, 15 August 2018 (2018-08-15), US, pages 1884 - 1893, XP055707585, ISSN: 0887-624X, DOI: 10.1002/pola.29076
- KAN ZHANG ET AL: "High performance crosslinked polyimide based on main-chain type polybenzoxazine", RSC ADVANCES, vol. 4, no. 107, 1 January 2014 (2014-01-01), pages 62550 - 62556, XP055707590, ISSN: 2046-2069, DOI: 10.1039/C4RA12015A
- HIROAKI OIE ET AL: "Synthesis of networked polymers by crosslinking reactions of polybenzoxazine bearing allyl group in the side chain", JOURNAL OF POLYMER SCIENCE, PART A: POLYMER CHEMISTRY, vol. 51, no. 9, 1 May 2013 (2013-05-01), US, pages 2035 - 2039, XP055707593, ISSN: 0887-624X, DOI: 10.1002/pola.26590
- TAREK AGAG ET AL: "Crosslinked polyamide based on main-chain type polybenzoxazines derived from a primary amine-functionalized benzoxazine monomer", JOURNAL OF POLYMER SCIENCE, PART A: POLYMER CHEMISTRY, vol. 49, no. 20, 15 October 2011 (2011-10-15), US, pages 4335 - 4342, XP055707599, ISSN: 0887-624X, DOI: 10.1002/pola.24867
- THANYALAK CHAISUWAN ET AL: "High-performance maleimide and nitrile-functionalized benzoxazines with good processibility for advanced composites applications", JOURNAL OF APPLIED POLYMER SCIENCE, vol. 101, no. 1, 1 January 2006 (2006-01-01), pages 548 - 558, XP055063540, ISSN: 0021-8995, DOI: 10.1002/app.23509

## Description

### Domaine Technique

L'invention concerne un procédé de fabrication d'un produit réticulé ayant des propriétés de charbonnement et de stabilité thermique améliorées. Ce produit réticulé peut notamment être utilisé en tant que résine ablative pour la constitution d'une tuyère de propulseur ou d'un corps de rentrée atmosphérique.

### Technique antérieure

Il est connu de réaliser des protections thermiques ablatives de tuyères de propulseurs ou de corps de rentrée atmosphérique à partir de résines phénoliques qui sont synthétisées à partir de formaldéhyde et de phénol, afin d'obtenir des densités d'aromatiques et de réticulation élevées, et donc un taux de coke important. Le formaldéhyde et le phénol sont toutefois des composés classés Cancérigène Mutagène Reprotoxique (CMR) de catégorie 1B et 2 dont il serait souhaitable de limiter l'usage, notamment pour anticiper d'éventuelles interdictions en Union Européenne. En outre, la réaction du phénol et du formaldéhyde est une polycondensation pendant laquelle de l'eau est produite. Cette eau peut se retrouver piégée au sein du produit fini, conduisant à une baisse des performances. Dans l'optique de résoudre ce problème, des développements ont été poursuivis afin d'obtenir des résines ablatives par réticulation de benzoxazines. Ces développements réduisent le piégeage de l'eau dans le produit fini mais les solutions de la littérature, fournissant un produit fini avec des propriétés de stabilité thermique et de charbonnement compatibles d'une application en tant que résine ablative, mettent en jeu des benzoxazines obtenues par réaction du phénol avec le formaldéhyde et une amine, et se basent donc sur la mise en œuvre de composés classés CMR. De plus, lorsque ces benzoxazines sont synthétisées sans phénol, elles présentent des propriétés de stabilité thermique et de charbonnement peu performantes.

Il serait souhaitable de disposer d'une nouvelle voie de synthèse de matériaux présentant des propriétés de stabilité thermique et de charbonnement adaptées à la réalisation de protections thermiques ablatives pour tuyères de propulseurs ou corps de rentrée atmosphérique.

### Exposé de l'invention

L'invention fournit un procédé de fabrication d'un produit réticulé comprenant la réticulation d'un monomère benzoxazine de formule A fournie ci-dessous : 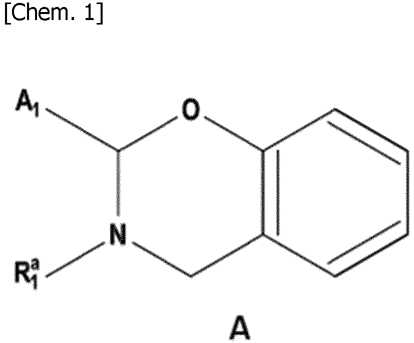 dans cette formule :
A₁ est un groupe carbocyclique aromatique ou hétérocyclique aromatique, monocyclique ou polycyclique, substitué par un groupement réticulable, ledit groupement réticulable est choisi parmi : un groupement hydroxyle, un groupement carbonitrile, un groupement phthalonitrile,
R₁^{a} est choisi parmi : les groupes furfuryles, substitués ou non substitués.

L'invention propose l'emploi de monomères benzoxazine ayant un groupement réticulable permettant d'obtenir, après réticulation, un produit ayant de hautes propriétés de stabilité thermique et de charbonnement, compatibles d'une application en tant que résine ablative pour la fabrication d'une tuyère de propulseur ou d'un corps de rentrée atmosphérique. L'invention fournit une nouvelle voie de synthèse de résines ablatives limitant l'utilisation de composés CMR, et s'affranchissant en particulier de l'emploi de formaldéhyde.

A₁ est un groupe carbocyclique aromatique ou hétérocyclique aromatique, monocyclique ou polycyclique, substitué par le groupement réticulable.

Le choix de tels groupements permet avantageusement d'obtenir après réticulation du monomère benzoxazine un produit présentant des propriétés de stabilité thermique et de charbonnement améliorées.

En particulier, A₁ peut être un cycle benzénique substitué par le groupement réticulable. Selon un exemple et quel que soit le mode de réalisation considéré, le groupement réticulable est un groupement hydroxyle.

R₁^{a} est choisi parmi : les groupes furfuryles, substitués ou non substitués.

Le choix de tels groupements permet avantageusement d'obtenir après réticulation du monomère benzoxazine un produit présentant des propriétés de stabilité thermique et de charbonnement améliorées.

La présence de l'oxygène du cycle furfuryle permet, en acceptant les liaisons hydrogène, d'apporter une stabilisation accrue du réseau réticulé, et d'augmenter ainsi davantage encore le taux de charbonnement.

Selon un exemple, le procédé comprend, avant la réticulation du monomère benzoxazine de formule A, la condensation d'une amine de formule A11 avec un aldéhyde de formule A12 afin d'obtenir le monomère benzoxazine de formule A, les formules A11 et A12 étant fournies ci-dessous :

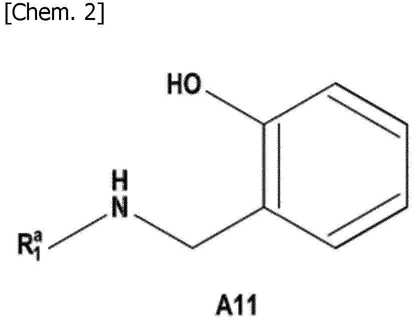

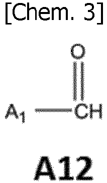

En particulier, le procédé peut comprendre, avant la condensation, l'obtention de l'amine de formule A11, cette obtention comprenant :
- une réaction d'addition d'une amine de formule A21 sur un aldéhyde de formule A22 afin de former une imine de formule A23, et
- une réaction de réduction de l'imine de formule A23 en amine de formule A11,
les formules A21, A22 et A23 étant fournies ci-dessous:

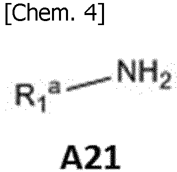

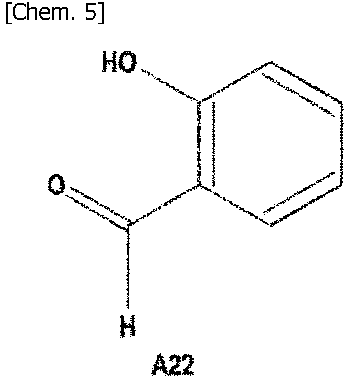

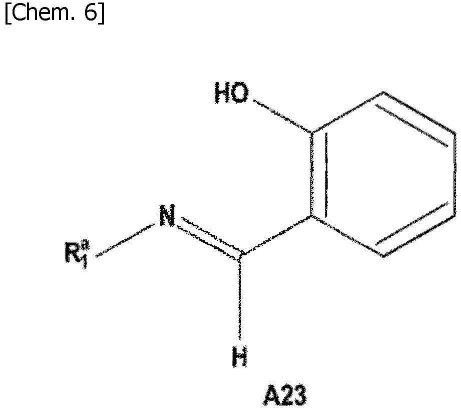

L'amine de formule A21 est par exemple choisie parmi les composés suivants : la furfurylamine.

L'aldéhyde de formule A22 est le salicylaldéhyde. L'aldéhyde de formule A22 est un monoaldéhyde, c'est-à-dire ne comprend qu'une seule fonction aldéhyde.

L'aldéhyde de formule A12 est par exemple choisi parmi les composés suivants : hydroxybenzaldéhyde, par exemple para-hydroxybenzaldéhyde, vanilline, syringaldéhyde ou phthalonitrile-benzaldéhyde, par exemple para-phthalonitrile-benzaldéhyde.

Dans un autre exemple, le procédé comprend, avant la réticulation du monomère benzoxazine de formule A :
- la condensation de l'amine de formule A11 avec un aldéhyde de formule A13 afin d'obtenir un précurseur benzoxazine de formule A14, la formule A11 étant définie ci-dessus et les formules A13 et A14 étant fournies ci-dessous : 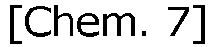 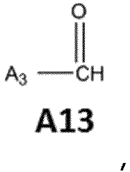 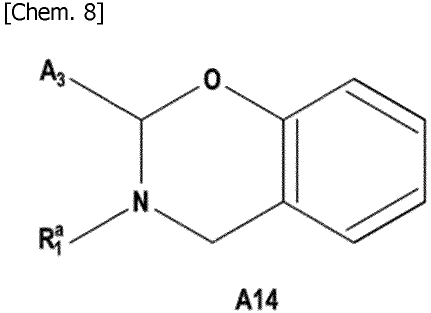 où A₃ désigne un groupement précurseur permettant d'obtenir la fonction A₁ portant le groupement réticulable après une réaction de fonctionnalisation, et
- la réalisation de la réaction de fonctionnalisation du précurseur benzoxazine de formule A14 afin d'obtenir le monomère benzoxazine de formule A.

L'aldéhyde de formule A13 est par exemple choisi parmi les composés suivants : hydroxybenzaldéhyde, par exemple para-hydroxybenzaldéhyde, vanilline, syringaldéhyde, aminobenzaldéhyde, par exemple para-aminobenzaldéhyde.

Les groupements hydroxyles et amines peuvent être des groupements réticulables comme décrit plus haut ou les groupements hydroxyles et amines peuvent constituer des groupements précurseurs d'autres groupements réticulables.

Quel que soit l'exemple considéré, la condensation peut être réalisée en portant le mélange de l'amine et de l'aldéhyde au reflux. On peut réaliser la condensation dans le toluène, le méthanol, l'éthanol ou encore sans solvant.

L'invention vise également une tuyère de propulseur obtenue en mettant en œuvre un produit réticulé obtenu par le procédé décrit plus haut. L'invention concerne également un procédé de fabrication d'une tuyère de propulseur dans lequel la tuyère est fabriquée en mettant en œuvre un produit réticulé obtenu par le procédé décrit plus haut.

La tuyère de propulseur peut être en matériau composite. Dans ce cas, la fabrication de la tuyère peut comporter une première étape de formation d'une préforme fibreuse de la tuyère à obtenir imprégnée par le monomère benzoxazine de formule A décrit plus haut. Cette fabrication peut en outre comporter une deuxième étape traitement thermique de la préforme fibreuse imprégnée de manière à réticuler le monomère benzoxazine de formule A et obtenir la tuyère de propulseur.

La préforme fibreuse peut par exemple comporter des fibres de carbone, de silice, de verre ou d'un matériau céramique, par exemple de carbure de silicium. La préforme fibreuse destinée à former le renfort fibreux de la tuyère peut être formée de diverses manières (drapage de couches de tissu pré-imprégnées, par exemple). On peut ainsi en particulier draper ou bobiner des couches de tissu bi-dimensionnel ou tri-dimensionnel imprégnées sur une forme ayant une surface reproduisant la géométrie désirée d'une surface interne ou externe de la tuyère à réaliser afin d'obtenir la préforme imprégnée.

En variante, on peut d'abord obtenir la préforme fibreuse de la tuyère à obtenir puis placer cette préforme dans une cavité d'injection et injecter ensuite le monomère benzoxazine de formule A dans la cavité de manière à imprégner la préforme. Dans ce cas, on peut mettre en œuvre une technique de moulage par transfert de résine (« Resin Transfer Molding ») pour imprégner la préforme fibreuse.

La réticulation peut être effectuée en imposant une température supérieure ou égale à 130°C, par exemple comprise entre 180°C et 250°C.

En variante, l'invention vise également un corps de rentrée atmosphérique obtenu en mettant en œuvre un produit réticulé obtenu par le procédé décrit plus haut. L'invention concerne également un procédé de fabrication d'un corps de rentrée atmosphérique dans lequel le corps de rentrée atmosphérique est fabriqué en mettant en œuvre un produit réticulé obtenu par le procédé décrit plus haut.

Quel que soit le mode de réalisation considéré, la réticulation mise en œuvre pour obtenir le produit réticulé peut être une homo-réticulation du monomère benzoxazine de formule A sur lui-même.

### Brève description des dessins

[Fig. 1] La figure 1 est un thermogramme obtenu par calorimétrie différentielle à balayage (« Differential Scanning Calorimetry » ; « DSC ») d'un premier exemple de monomère benzoxazine selon l'invention.
[Fig. 2] La figure 2 est un résultat d'analyse thermogravimétrique (« thermogravimetric analysis » ; « TGA ») d'un produit réticulé obtenu par réticulation de ce premier exemple de monomère benzoxazine.
[Fig. 3] La figure 3 est un thermogramme DSC d'un deuxième exemple de monomère benzoxazine selon l'invention.
[Fig. 4] La figure 4 est un résultat d'analyse thermogravimétrique d'un produit réticulé obtenu par réticulation de ce deuxième exemple de monomère benzoxazine.

### Description des modes de réalisation

### Exemples

### Exemple 1 : synthèse d'un monomère benzoxazine à partir de furfurylaminométhylphénol et para-hydroxybenzaldéhyde puis réticulation subséquente

On fait réagir la furfurylamine avec le salicylaldéhyde dans des proportions stœchiométriques dans le méthanol au reflux pendant 2h, dans le but de former l'imine correspondante. L'imine est réduite en amine avec 1 équivalent de NaBH₄ ajouté à 0°C dans une solution de l'imine dans le MeOH, puis on réalise un chauffage à reflux pendant 2 heures. Le furfurylaminométhylphénol ainsi synthétisé est dissous dans le toluène avec 1 équivalent de para-hydroxybenzaldéhyde, puis porté au reflux dans un appareil Dean Stark, afin de retirer l'eau générée pendant la réaction de condensation. La réaction est arrêtée lorsque la conversion des aldéhydes a atteint son maximum, suivi par RMN du proton. Après évaporation du solvant sous pression réduite, la benzoxazine isolée est un solide jaune pâle. Le produit a été caractérisé par RMN et spectroscopie infrarouge et la structure a été confirmée.

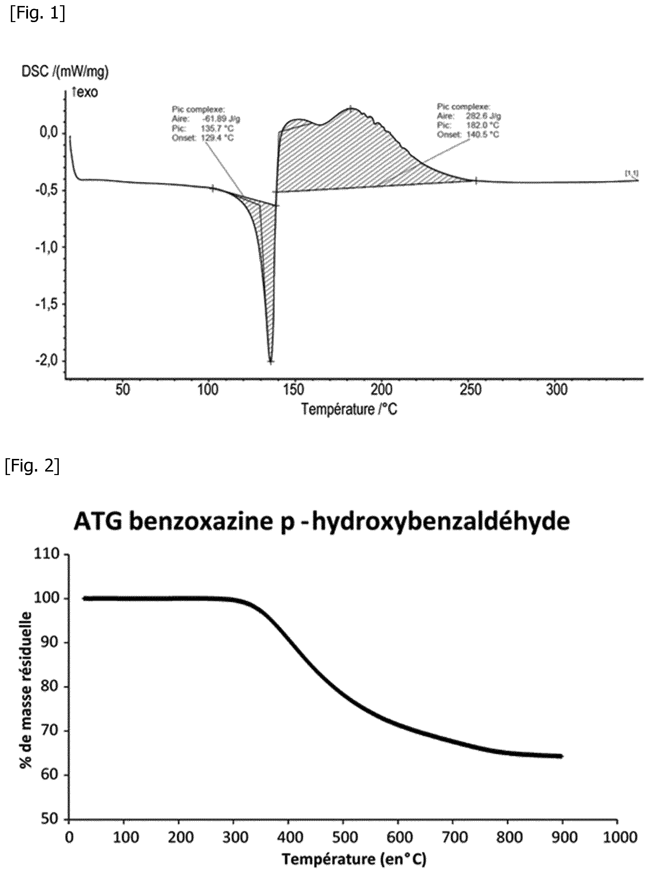

La caractérisation thermique par DSC a révélé une température de fusion de 130°C ainsi qu'une réaction exothermique entre 140°C et 250°C correspondant à la réticulation, représentant 283J/g d'enthalpie par rapport à la référence, avec une rampe de 10°C/min dans des creusets en acier scellés haute pression. Le thermogramme DSC obtenu est fourni à la figure 1.

L'analyse thermogravimétrique a montré un taux de coke après polymérisation *in situ* de 64% sous atmosphère d'azote, ainsi qu'une température de dégradation de 10% de la masse totale de 402°C (rampe de chauffe: 10°C/min). Cela montre une excellente stabilité thermique pour cette résine. Le graphe d'analyse thermogravimétrique obtenu est fourni à la figure 2.

### Exemple 2 : synthèse d'un monomère benzoxazine à partir de furfurylaminométhylphénol et para-phthalonitrile-benzaldéhyde puis réticulation subséquente

On fait réagir le para-hydroxybenzaldéhyde avec 1 équivalent de K₂CO₃ dans le diméthylformamide, à 0°C. On ajoute progressivement 1,1 équivalent de 4-nitrophthalonitrile à 0°C. On laisse sous agitation à température ambiante pendant une nuit. On précipite ensuite le milieu réactionnel dans 50 fois le volume d'eau glacée. Le milieu hétérogène d'une couleur jaune est ensuite filtré sous vide, et on obtient un solide visqueux blanc cassé. Ce solide est dissout dans le dichlorométhane, et lavé 3 fois avec une solution saturée de chlorure de sodium. La phase organique est isolée, séchée sur sulfate de magnésium et le produit récupéré par évaporation du solvant sous pression réduite. Le produit est une poudre de couleur blanc cassé.

On fait réagir la furfurylamine avec le salicylaldéhyde dans des proportions stœchiométriques dans le méthanol au reflux pendant 2h, dans le but de former l'imine correspondante. L'imine est réduite en amine avec 1 équivalent de NaBH₄ ajouté à 0°C dans une solution de l'imine dans le MeOH, puis on réalise un chauffage à reflux pendant 2 heures. Le furfurylaminométhylphénol ainsi synthétisé est dissous dans le toluène avec 1 équivalent de para-phthalonitrile-benzaldéhyde, puis porté au reflux dans un appareil Dean Stark, afin de retirer l'eau générée pendant la réaction de condensation. La réaction est arrêtée lorsque la conversion des aldéhydes a atteint son maximum, suivi par RMN du proton. Après évaporation du solvant sous pression réduite, la benzoxazine isolée est un solide visqueux blanc cassé. Le produit a été caractérisé par RMN et la structure a été confirmée.

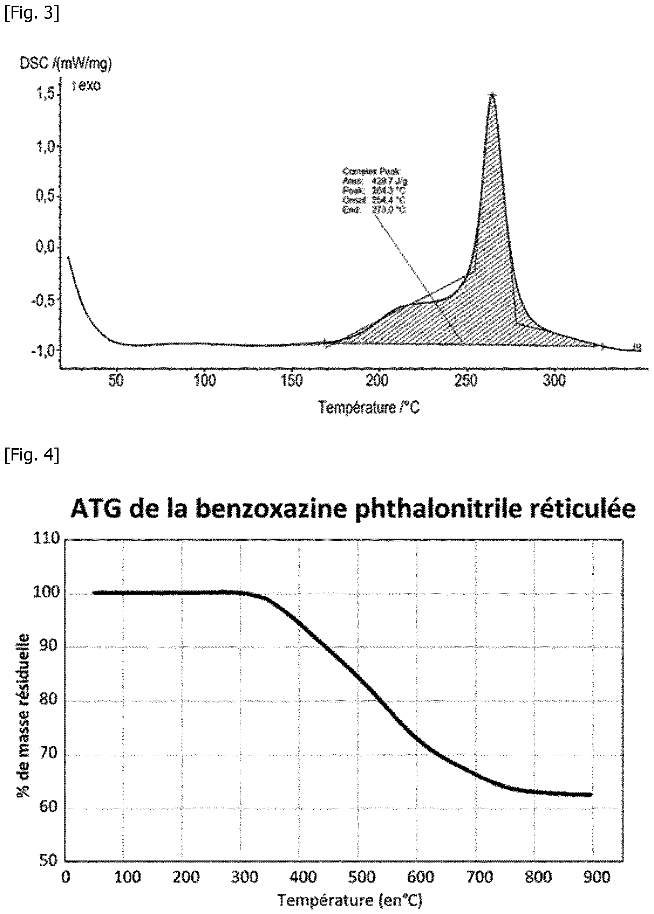

La caractérisation thermique par DSC a révélé une réaction exothermique entre 170°C et 278°C, représentant 430J/g d'enthalpie par rapport à la référence, avec une rampe de 10°C/min dans des creusets en acier scellés haute pression. Le thermogramme DSC obtenu est fourni à la figure 3.

L'analyse thermogravimétrique a montré un taux de coke après polymérisation *in situ* de 62% sous atmosphère d'azote, ainsi qu'une température de dégradation de 10% de la masse totale de 444°C (rampe de chauffe: 10°C/min). Le graphe d'analyse thermogravimétrique obtenu est fourni à la figure 4.

## Revendications

1. Procédé de fabrication d'un produit réticulé comprenant la réticulation d'un monomère benzoxazine de formule A fournie ci-dessous : 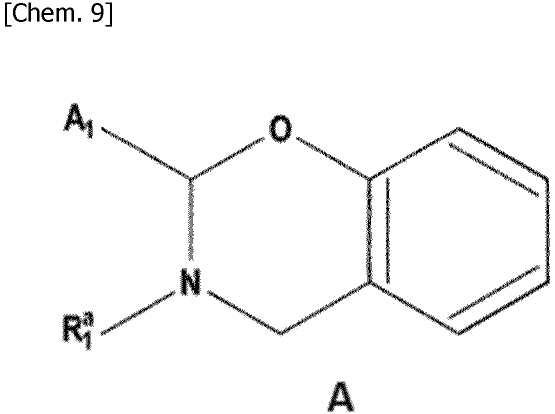 dans cette formule :
A₁ est un groupe carbocyclique aromatique ou hétérocyclique aromatique, monocyclique ou polycyclique, substitué par un groupement réticulable, ledit groupement réticulable est choisi parmi : un groupement hydroxyle, un groupement carbonitrile, un groupement phthalonitrile,
R₁^{a} est choisi parmi : les groupes furfuryles, substitués ou non substitués.

2. Procédé selon la revendication 1, dans lequel A₁ est un cycle benzénique substitué par le groupement réticulable.

3. Procédé selon l'une quelconque des revendications 1 ou 2, dans lequel le groupement réticulable est un groupement hydroxyle.

4. Tuyère de propulseur obtenue en mettant en œuvre un produit réticulé obtenu par le procédé selon l'une quelconque des revendications 1 à 3.

5. Corps de rentrée atmosphérique obtenu en mettant en œuvre un produit réticulé obtenu par le procédé selon l'une quelconque des revendications 1 à 3.

## Patentansprüche

1. Verfahren zur Herstellung eines vernetzten Produkts, umfassend das Vernetzen eines Benzoxazinmonomers der folgenden Formel A: 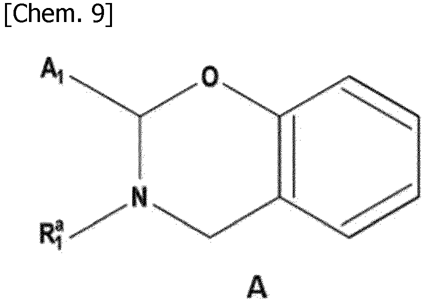 wobei in dieser Formel:
A₁ eine monocyclische oder polycyclische aromatische heterocyclische oder aromatische carbocyclische Gruppe ist, substituiert durch eine vernetzbare Gruppe, wobei die vernetzbare Gruppe ausgewählt ist aus: einer Hydroxylgruppe, einer Carbonitrilgruppe, einer Phthalonitrilgruppe,
R₁^{a} ausgewählt ist aus: den substituierten oder unsubstituierten Furfurylgruppen.

2. Verfahren nach Anspruch 1, wobei A₁ ein Benzolring ist, der durch die vernetzbare Gruppe substituiert ist.

3. Verfahren nach einem der Ansprüche 1 oder 2, wobei die vernetzbare Gruppe eine Hydroxylgruppe ist.

4. Treibmitteldüse, erhalten durch Umsetzen eines vernetzten Produkts, das durch das Verfahren nach einem der Ansprüche 1 bis 3 erhalten wurde.

5. Wiedereintrittskörper in die Atmosphäre, der durch Umsetzen eines vernetzten Produkts hergestellt wird, das durch das Verfahren nach einem der Ansprüche 1 bis 3 erhalten wurde.

## Claims

1. A process for manufacturing a cross-linked product comprising the cross-linking of a benzoxazine monomer of formula A provided below: 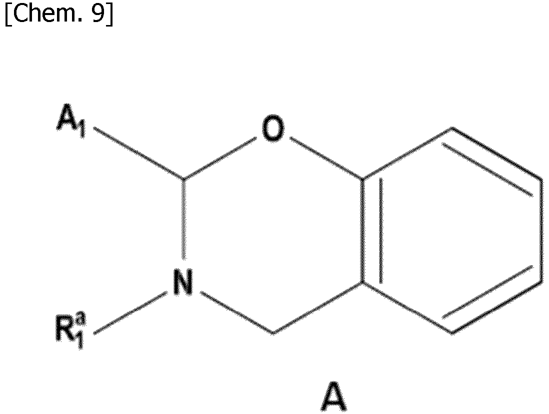 in this formula:
A₁ is selected from aromatic carbocyclic or heterocyclic, monocyclic or polycyclic, groups, substituted by a cross-linkable group, said cross-linkable group is selected from: a hydroxyl group, a carbonitrile group, a phthalonitrile group,
R₁^{a} is selected from: substituted or unsubstituted furfuryl groups.

2. The process as claimed in claim 1, wherein A₁ is a benzene ring substituted by the cross-linkable group.

3. The process as claimed in claim 1 or 2, wherein the cross-linkable group is a hydroxyl group.

4. A rocket nozzle obtained by using a cross-linked product obtained by the process as claimed in any one of claims 1 to 3.

5. A re-entry vehicle obtained by using a cross-linked product obtained by the process as claimed in any one of claims 1 to 3.
